# EUROPÄISCHE PATENTSCHRIFT

(11) **EP 2 922 810 B1**
(45) Veröffentlichungstag und Bekanntmachung des Hinweises auf die Patenterteilung: **21.12.2016**
(21) Anmeldenummer: 13799231.9
(22) Anmeldetag: 19.11.2013
(51) Int. Cl.: C07C 27/00, C07C 68/06, C07C 69/96, C07C 29/128, C07C 31/20

(54) **VERFAHREN ZUR HERSTELLUNG VON DIALKYLCARBONATEN**
PROCESS FOR PRODUCING DIALKYL CARBONATES
PROCÉDÉ DE PRÉPARATION DE CARBONATES DE DIALKYLE

(30) Priorität: 21.11.2012 EP 12193568
(43) Veröffentlichungstag der Anmeldung: 30.09.2015
(73) Patentinhaber: Covestro Deutschland AG, 51373 Leverkusen (DE)
(72) Erfinder: OOMS, Pieter, 47800 Krefeld (DE); RISSE, Friedhelm, 50739 Köln (DE); DÜX, Andre, 53332 Bornheim (DE); BUCHALY, Carsten, 40239 Düsseldorf (DE); PANCUR, Thomas, 24161 Altenholz (DE); SUSANTO, Arthur, 50670 Köln (DE); RONGE, Georg, Singapore 049514 (SG); VANDEN EYNDE, Johan, B-9052 Zwijnaarde (BE); WUYTACK, Wim, B-9240 Zele (BE)
(74) Vertreter: Levpat
(86) Internationale Anmeldenummer: PCT/EP2013/074150
(87) Internationale Veröffentlichungsnummer: WO 2014/079835

(56) Entgegenhaltungen:
- EP-A2- 2 322 261
- EP-A2- 2 354 115
- WO-A1-2007/096343

## Beschreibung

Die vorliegende Erfindung betrifft ein kontinuierliches Verfahren zur Herstellung von niederen Dialkylcarbonaten als Hauptprodukt und Alkylenglykol als Nebenprodukt durch Umesterung eines zyklischen Alkylencarbonats (z.B. Ethylen- oder Propylencarbonat) mit niederen Alkylalkoholen in Gegenwart eines Katalysators sowie die erforderliche Aufreinigung des Dialkylcarbonats in einem nachfolgenden Verfahrensschritt. Zur Optimierung der Wirtschaftlichkeit und Energieeffizienz des Verfahrens werden zusätzliche Vorrichtungen zur Zwischenerhitzung der apparateintemen Flüssigkeitströme eingesetzt.

Die Herstellung von Dialkylcarbonaten aus zyklischem Alkylencarbonat und Alkylalkohol, bei der gleichzeitig Alkylenglykol als Nebenprodukt entsteht, ist bekannt und vielfach beschrieben worden. In US-6,930,195 B wurde diese katalysierte Umesterungsreaktion als zweistufige Gleichgewichtsreaktion beschrieben. In der ersten Reaktionsstufe reagiert das zyklische Alkylencarbonat mit Alkylalkohol zu Hydroxy-alkylcarbonat als Zwischenprodukt. Das Zwischenprodukt wird dann in der zweiten Reaktionsstufe mit Hilfe von Alkylalkohol umgesetzt zu den Produkten: Dialkylcarbonat und Alkylenglykol.

Für die technische Realisierung des Dialkylcarbonat-Herstellungsverfahrens hat sich die Verwendung einer reaktiven Destillationskolonne (im Folgenden auch Umesterungskolonne genannt), die unter anderem bereits in EP 530 615 A, EP 569 812 A und EP 1 086 940 A beschrieben wurde, als besonders günstig erwiesen. In EP 569 812 A wird das zyklische Alkylencarbonat in den oberen Teil der Umesterungskolonne und der Dialkylcarbonat enthaltende Allylalkohol in den mittleren oder unteren Teil der Umesterungskolonne kontinuierlich eingeführt. Zusätzlich wird unterhalb der Einführung des Dialkylcarbonat enthaltenden Alkylalkohols nahezu reiner Alkylalkohol eingeführt. Das Schwersiedergemisch, welches das hergestellte Alkylenglykol als Nebenprodukt beinhaltet, wird am Sumpf der Umesterungskolonne kontinuierlich abgezogen. Das Leichtsiedergemisch, welches das hergestellte Dialkylcarbonat beinhaltet, wird am Kopf der Umesterungskolonne als Dialkylcarbonat-Alkylalkohol-Gemisch abgezogen und einem weiteren Aufreinigungsschritt unterworfen.

Die Destillationskolonne für die Aufreinigung des Dialkylcarbonat-Alkylalkohol-Gemisches wird bei einem höheren Druck als dem in der Umesterungskolonne herrschenden betrieben, so dass ein weiteres Dialkylcarbonat-Alkylalkohol-Gemisch mit einem niedrigeren Dialkylcarbonat-Anteil am Kolonnenkopf dieser Destillationskolonne abgezogen werden kann. Das Dialkylcarbonat als Hauptprodukt wird am Sumpf dieser Aufreinigungskolonne mit hoher Reinheit gewonnen.

Für die Entwicklung eines wirtschaftlich attraktiven Herstellungsverfahrens für Dialkylcarbonate spielen viele Faktoren eine wichtige Rolle. Die meisten Literaturquellen beschäftigen sich mit den Reaktionsparametern, wie z.B. Umsatz, Selektivität oder auch Produktreinheit. Seltener wurde die Energieeffizienz des Verfahrens thematisiert (z.B. in EP 569 812 A, JP 2003-104937, WO 2007/096340, WO 2007/096343), obwohl diese Faktoren nicht unerheblich zu der wirtschaftlichen Attraktivität des Verfahrens beitragen. Daher werden in dieser Erfindung Maßnahmen eingeführt, um die Energieeffizienz des Verfahrens zu erhöhen.

In EP 569 812 A wird der Energieeinsatz in der Herstellung des Dialkylcarbonates dadurch reduziert, dass viele verfahrensinterne Ströme nicht kondensiert sondern als dampfförmige Ströme geführt werden.

In WO 2007/096340 wird ein Verfahren beschrieben, in dem Alkylencarbonat aus Alkylenoxid und CO₂ erzeugt und anschließend das Alkylencarbonat mit Alkylalkohol zu Dialkylcarbonat und Alkylenglykol umgesetzt wird, wobei das im zweiten Schritt entstehende Gemisch, welches Dialkylcarbonat und Alkylenglykol enthält, aufgereinigt wird. Die Reaktion zum Alkylencarbonat ist exotherm und der entsprechende Alkylencarbonat-Produktstrom wird zur Aufwärmung des Dialkylcarbonat-Alkylenglykol-Produktstromes in der Aufreinigung eingesetzt.

In WO 2007/096343 wird das in einer Umesterungskolonne aus Alkylencarbonat und Alkylalkohol entstehende Gemisch aus Dialkylcarbonat und Alkylalkohol mittels Extraktivdestillation gereinigt, wobei Alkylencarbonat als Extraktionsmittel dient. Nachdem das Dialkylcarbonat destillativ vom Extraktionsmittel getrennt wurde, wird mit dem heißen Sumpfablauf dieser Kolonne, der das Extraktionsmittel enthält, der der Umesterungskolonne zugeführte Alkylalkohol erwärmt.

In JP 2003-104937 werden verschiedene Verfahrensvarianten zur Aufarbeitung eines Ethylencarbonat-Ethylenglykol-Gemisches und Bereitstellung des gereinigten Ethylencarbonates für das Verfahren zur Herstellung von Dimethylcarbonat auch unter dem Gesichtspunkt des Energieverbrauchs betrachtet.

Die EP 2 354 115 A2 offenbart ein Verfahren zur Herstellung von Dialkylcarbonaten, wobei ein Partialkondensator den Dampfstrom am Kopf der Dialkylcarbonat-Aufreinigungskolonne kondensiert und dabei die Wärmeleistung, die zur Dampferzeugung genutzt werden kann, abgeführt wird. Die am Kopf der Dialkylcarbonat-Aufreinigungskolonne gewonnene Dampfmenge kann zur Vorerhitzung des Feeds dieser Kolonne und für die Verdampfung des Zustroms zur Umesterungskolonne genutzt werden. Die Aufreinigung von Alkylenglykol wir hier jedoch nicht erwähnt.

Keine der genannten Schriften beschreibt aber Verfahren oder Vorgehensweisen, wie unter Beibehaltung der Qualität des Hauptproduktes (Dialkylcarbonat) sowie des Nebenproduktes (Akylenglykol) die Umsetzung von Alkylencarbonat mit Alkylalkohol in der Umesterungskolonne besonders energieeffizient durchgeführt werden kann. Daher werden in dieser Erfindung Maßnahmen eingeführt, um die Energieeffizienz in diesem Verfahrensschritt zu erhöhen.

Es bestand daher der Bedarf an einem Verfahren, das zum einen eine höhere Energieeffizienz in der Umesterungskolonne aufweist und zum anderen die Qualität des Dialkylcarbonates und des Alkylenglykols nur unwesentlich beeinflusst.

Aufgrund des günstigen Einflusses auf das Temperaturprofil in der Umesterungskolonne und somit auch auf den Reaktionsumsatz werden die Fahrweisen bevorzugt, bei denen sowohl der nahezu reine als auch der Dialkylcarbonat enthaltende Alkylalkoholstrom der Umesterungskolonne gasförmig zugeführt werden. Diese Fahrweisen tragen auch dazu bei, den Energiebedarf des Sumpfverdampfers der Umesterungskolonne zu reduzieren. Zur Verdampfung der beiden Ströme wird vorzugsweise niederstufiger Dampf oder heißes Kreislaufwasser auf einem Temperaturniveau größer T_{V} eingesetzt.

Es wurde nun herausgefunden, dass die Energieeffizienz dadurch erhöht werden kann, dass der Bedarf an externer, d.h. außerhalb des vorliegenden Verfahrens erzeugter, Wärmeenergie reduziert wird, welche zum Betrieb des bzw. der Wärmetauscher zur Verdampfung der Alkylalkoholströme benötigt wird, indem besonders einfach und günstig bei gleichbleibender Produktqualität die Kondensationsenergie an der Kolonne zur Aufreinigung des Alkylenglykols wiedergewonnen und den Wärmetauschern zur Verdampfung der Alkylalkoholströme direkt oder indirekt zugeführt wird.

Die am Kopfkondensator der Alkylenglykolaufreinigungskolonne gewonnene Wärmeenergie oder auch die in anderen chemischen Herstellverfahren gewonnene Wärmeenergie auf dem Temperaturniveau T_{K} kann entweder direkt oder indirekt dem oder den Wärmetauschern zur Verdampfung der Alkylalkoholströme zugeführt werden. Bei direkter Zuführung erwärmt bzw. verdampft der Stoffstrom, der kondensiert oder abgekühlt werden soll, mittels des oder der Wärmetauscher den bzw. die der Umesterungskolonne zugeführten Alkylalkoholströme. Bei indirekter Zuführung erwärmt der zu kondensierende oder abzukühlende Stoffstrom über die Vermittlung eines oder mehrerer Wärmeträgermedien die der Umesterungskolonne zugeführten Alkylalkoholenthaltenden Ströme. Als Wärmeträgermedien kommen Gase, Dämpfe oder Flüssigkeiten in Frage, bevorzugt dampfförmige oder flüssige technische Wärmeträgermedien wie beispielsweise Wasser, Wärmeträger auf Mineralölbasis oder synthetische Wärmeträger (z.B. Diphyl™, Marlotherm®).

Besonders bevorzugte Wärmeträgermedien sind Wasser oder Wasserdampf. In den nachfolgenden Prozessstufen zur Aufarbeitung des in der Umesterungskolonne hergestellten Alkylenglykols entsteht viel Abwärme, die beispielsweise im jeweiligen Kopfkondensator der Aufarbeitungskolonnen abgeführt werden muss. Diese Wärme wird üblicherweise nicht genutzt, weil entweder die Temperaturstufe zu niedrig oder die Wärmemenge zu gering ist.

Es wurde überraschenderweise herausgefunden, dass in der letzten Aufarbeitungsstufe zur Herstellung von Alkylenglykol Abwärme bei ausreichender Temperaturstufe und in ausreichender Menge anfällt, um in einem anderen Prozessteil eingesetzt werden zu können. Besonders bevorzugt wird diese Abwärme aus dem Kopfkondensator der Alkylenglykolaufarbeitungskolonne gewonnen. Diese Abwärme kann beispielsweise zur Vorerhitzung des Feeds in der Dialkylcarbonataufarbeitungskolonne oder zur Verdampfung des Alkohols für die Umesterungskolonne verwendet werden. Besonders bevorzugt wird die Abwärme zur Verdampfung des nahezu reinen Alkylalkoholstromes und/oder des Dialkylcarbonat enthaltenden Alkylalkoholstromes, der bzw. die der Umesterungskolonne zugeführt werden, eingesetzt.

Durch die Verringerung des Verbrauchs an externer Wärmeenergie unter gleichzeitiger Beibehaltung der hohen Produktqualität ergibt sich durch das erfindungsgemäße Verfahren ein signifikanter ökonomischer Vorteil.

Die oben genannte Wärmeintegration kann beispielsweise durch direkte Wärmeverschaltung in einem gemeinsamen Wärmetauscher oder durch indirekte Wärmeverschaltung mit Hilfe einer Kreislaufflüssigkeit (z.B. Wasser) als Wärmeträger realisiert werden. Durch die Nutzung der Abwärme aus der Alkylenglykolaufarbeitungskolonne ergibt sich durch das erfindungsgemäße Verfahren ein signifikanter ökönomischer Vorteil.

Im Rahmen der Erfindung gereinigte Dialkylcarbonate sind bevorzugt solche der allgemeinen Formel (I) 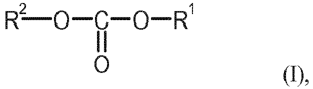 wobei R¹ und R² unabhängig voneinander für C₁-C₄-Alkyl stehen. Dabei können R¹ und R² gleich oder verschieden sein. Bevorzugt sind R¹ und R² gleich.

**C₁-C₄-Alkyl** steht im Rahmen der Erfindung beispielsweise für Methyl, Ethyl, n-Propyl, iso-Propyl, n-Butyl, sec.-Butyl, tert.-Butyl, **C₁-C₆-Alkyl** darüber hinaus beispielsweise für n-Pentyl, 1-Methylbutyl, 2-Methylbutyl, 3-Methylbutyl, neo-Pentyl, 1-Ethylpropyl, Cyclohexyl, Cyclopentyl, n-Hexyl, 1,1-Dimethylpropyl, 1,2-Dimethylpropyl, 1,2-Dimethylpropyl, 1-Methylpentyl, 2-Methylpentyl, 3-Methylpentyl, 4-Methylpentyl, 1,1-Dimethylbutyl, 1,2-Dimethylbutyl, 1,3-Dimethylbutyl, 2,2-Dimethylbutyl, 2,3-Dimethylbutyl, 3,3-Dimethylbutyl, 1-Ethylbutyl, 2-Ethylbutyl, 1,1,2-Trimethylpropyl, 1,2,2-Trimethylpropyl, 1-Ethyl-1-methylpropyl, 1-Ethyl-2-methylpropyl oder 1-Ethyl-2-methylpropyl, **C₁-C₃₄-Alkyl** darüber hinaus beispielsweise für n-Heptyl und n-Octyl, Pinakyl, Adamantyl, die isomeren Menthyle, n-Nonyl, n-Decyl, n-Dodecyl, n-Tridecyl, n-Tetradecyl, n-Hexadecyl oder n-Octadecyl. Gleiches gilt für den entsprechenden Alkylrest beispielsweise in Aralkyl- bzw. Alkylarylresten. Alkylenreste in den entsprechenden Hydroxyalkyl- oder Aralkyl- bzw. Alkylarylresten stehen beispielsweise für die den vorangehenden Alkylresten entsprechenden Alkylenreste.

Die vorangehenden Aufzählungen sind beispielhaft und nicht als Limitierung zu verstehen.

Bevorzugte Dialkylcarbonate sind Dimethylcarbonat, Diethylcarbonat, Di(n-propyl)carbonat, Di(iso-propyl)carbonat, Di(n-butyl)carbonat, Di(*sec*-butyl)carbonat oder Di(*tert*-butyl)carbonat. Besonders bevorzugt sind Dimethylcarbonat oder Diethylcarbonat. Ganz besonders bevorzugt ist Dimethylcarbonat.

Die Dialkylcarbonate werden bevorzugt aus zyklischen Alkylencarbonaten mit der Formel (II) hergestellt: 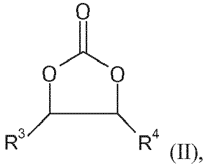 wobei in der Formel R³ und R⁴ gemeinsam mit den beiden Dreiring-C-Atomen ein C₂ - C₄-Alkylen darstellen. Bevorzugt wird als zyklisches Carbonat Ethylen- oder Propylencarbonat eingesetzt.

Die zyklischen Alkylencarbonate werden mit Alkylalkoholen der Form

R⁵-OH

umgesetzt, wobei R⁵ ein geradkettiges oder verzweigtes **C₁-C₄-Alkyl** bedeutet. Bevorzugt wird als Alkylalkohol Methanol oder Ethanol eingesetzt.

Zur Erzeugung der Dialkylcarbonate verwendete Umesterungskatalysatoren sind die dem Fachmann bekannt, beispielsweise Hydride, Oxide, Hydroxide, Alkoholate, Amide oder Salze von Alkalimetallen, wie Lithium, Natrium, Kalium, Rubidium und Cäsium, bevorzugt von Lithium, Natrium und Kalium, besonders bevorzugt von Natrium und Kalium (US 3,642,858 A, US 3 803 201 A, EP 1 082 A). Für den Fall des Einsatzes der Alkoholate können diese auch in situ durch Einsatz der elementaren Alkalimetalle und dem umzusetzenden Alkohol gebildet werden. Salze der Alkalimetalle können solche von organischen oder anorganischen Säuren sein, wie von Essigsäure, Propionsäure, Buttersäure, Benzoesäure, Stearinsäure, Kohlensäure (Carbonate oder Hydrogencarbonate), von Salzsäure, Bromwasserstoff- oder lodwasserstoffsäure, Salpetersäure, Schwefelsäure, Fluorwasserstoffsäure, Phosphorsäure, Blausäure, Rhodanwasserstoff, Borsäure, Zinnsäure, C₁-C₄-Stannonsäuren oder Antimonsäuren. In bevorzugter Weise kommen als Verbindungen der Alkalimetalle die Oxide, Hydroxide, Alkoholate, Acetate, Propionate, Benzoate, Carbonate und Hydrogencarbonate in Frage, in besonders bevorzugter Weise werden Hydroxide, Alkoholate, Acetate, Benzoate oder Carbonate eingesetzt. Solche Alkalimetallverbindungen (gegebenenfalls in situ gebildet aus den freien Alkalimetallen) werden in Mengen von 0,001 bis 2 Gew.-%, bevorzugt 0,003 bis 1,0 Gew.-%, besonders bevorzugt 0,005 bis 1,0 Gew.-%, bezogen auf das umzusetzende Reaktionsgemisch, eingesetzt.

Es ist möglich, solchen Alkalimetallverbindungen gegebenenfalls komplexierende Stoffe zuzusetzen. Beispielsweise seien genannt Kronenether wie Dibenzo-18-krone-6, Polyethylenglykole oder bicyclische stickstoffhaltige Kryptanden.

Solche Komplexiermittel werden in Mengen von 0,1 bis 200 mol-%, bevorzugt in 1 bis 100 mol-%, bezogen auf die Alkalimetallverbindung, eingesetzt.

Als Katalysatoren für die Herstellung von Dialkylcarbonate sind ferner Thallium-I- und Thallium-III-Verbindungen geeignet, wie die Oxide, Hydroxide, Carbonate, Acetate, Bromide, Chloride, Fluoride, Formiate, Nitrate, Cyanate, Stearate, Naphthenate, Benzoate, Cyclohexylphosphonate, Hexahydrobenzoate, das Cyclopentadienylthallium, Thalliummethylat, Thalliumethylat, bevorzugt Tl-(I)-oxid, Tl-(I)-hydroxid, Tl-(I)-carbonat, T1-(I)-acetat, Tl-(III)-acetat, Tl-(I)-fluorid, T1-(I)-formiat, Tl-(I)-nitrat, Tl-(I)-naphtenat und Tl-(I)-methylat (EP 1 083). Die Mengen an Thalliumkatalysator sind nicht besonders kritisch. Sie betragen im allgemeinen 0,0001-10 Gew.-%, bevorzugt 0,001-1 Gew.-%, bezogen auf das gesamte Reaktionsgemisch. Im Herstellungsverfahren können weiterhin stickstoffhaltige Basen als Katalysatoren eingesetzt werden (US 4 062 884). Genannt seien beispielsweise sek. oder tert. Amine wie Triethylamin, Tributylamin, Methyldibenzylamin, Dimethylcyclohexylamin u.a..

Die eingesetzten Mengen der stickstoffhaltigen Basen liegen von 0,01 bis 10 Gew.-%, bevorzugt von 0,1 bis 5 Gew.-%, besonders bevorzugt von 0,1 bis 1 Gew.-%, bezogen auf das gesamte Reaktionsgemisch. Als Katalysatoren sind ferner Verbindungen aus der Gruppe der Phosphine, Stibine, Arsine oder der zweiwertigen Schwefel- und Selen-Verbindungen sowie deren Oniumsalze einsetzbar (EP 180 387, US 4 734 519).

Beispielhaft seien die folgenden genannt: Tributylphosphin, Triphenylphosphin, Diphenylphosphin, 1,3-Bis-(diphenylphosphino)propane, Triphenylarsin, Trimethylarsin, Tributylarsin, 1,2-Bis-(diphenylarsino)ethan, Triphenylantimon, Diphenylsulfid, Diphenyldisulfid, Diphenylselenid, Tetraphenylphosphoniumhalogenid (Cl, Br, J), Tetraphenylarsoniumhalogenid (Cl, Br, J), Triphenylsulfoniumhalogenid (Cl, Br) usw.

Die bevorzugten Einsatzmengen dieser Katalysatorengruppe liegen im Bereich von 0,1 bis 10 Gew.-%, bevorzugt von 0,1 bis 5 Gew.-%, besonders bevorzugt im Bereich von 0,1 bis 2 Gew.-%, bezogen auf das gesamte Reaktionsgemisch.

Weiterhin als Katalysatoren einsetzbar sind Verbindungen des Zinns, Titans oder Zirkoniums (US-4,661,609 A). Beispiele solcher Systeme sind Butylstannonsäure, Zinnmethoxid, Dimethylzinn, Dibutylzinnoxid, Dibutylzinndilaurat, Tributylzinnhydrid, Tributylzinnchlorid, Zinn(II)ethyl-hexanoate, Zirkoniumalkoxide (Methyl, Ethyl, Butyl), Zirkonium(IV)halogenide (F, Cl, Br, J), Zirkoniumnitrate, Zirkoniumacetylacetonat, Titanalkoxide (Methyl, Ethyl, Isopropyl), Titanacetat, Titanacetylacetonat usw.

Die bevorzugt einsetzbaren Mengen dieser Katalysatoren betragen 0,1 bis 10 Gew.-%, bevorzugt 0,1 bis 5 Gew.-%, bezogen auf das Gesamtgemisch.

Im Herstellungsverfahren können weiterhin bifunktionelle Katalysatoren der Formel (III)

[AₐX_{b}]ₘ • [B_{c}Y_{d}]ₙ (III)

eingesetzt werden. In diesen bifunktionellen Katalysatoren wird das molare Verhältnis der beiden in eckigen Klammern stehenden Komponenten durch die Indizes m und n ausgedrückt. Diese Indizes können unabhängig voneinander Werte von 0,001-1, bevorzugt 0,01-1, besonders bevorzugt 0,05-1 und ganz besonders bevorzugt 0,1-1 annehmen. Innerhalb der eckigen Klammern stehen neutrale Salze aus je einem Kation und einem Anion. Die Indizes a und b stellen unabhängig voneinander ganze Zahlen von 1-5 dar; die Indizes C und d bedeuten unabhängig voneinander ganze Zahlen von 1-3, wobei den Forderungen der Valenzen der Kationen und Anionen zur Bildung solcher neutraler Salze zu entsprechen ist. Des Weiteren bedeuten in (III) A das Kation eines Metalles, das der dritten Periode und Gruppe IIa, der vierten Periode und Gruppe IIa, IVa-VIIIa, Ib oder IIb, der fünften Periode und Gruppe IIa, IVa-VIIa oder IVb bzw. der sechsten Periode und Gruppe IIa-VIa des Periodensystems der Elemente in der Kurzperiodenform angehört.

Die in Frage kommenden Metalle für das Kation A entnimmt der Fachmann den üblichen Darstellungen des Periodensystems der Elemente (Mendelejew) in der Kurzperiodenform. In bevorzugter Weise handelt es sich bei A um das Kation eines der Metalle Mg, Ca, Sr, Ba, Zn, Cu, Mn, Co, Ni, Fe, Cr, Mo, W, Ti, Zr, Sn, Hf, V und Ta, in bevorzugter Weise um das Kation eines der Metalle Mg, Ca, Zn, Co, Ni, Mn, Cu und Sn. Außer den nicht komplexierten Kationen der genannten Metalle kommen auch kationische Oxokomplexe der genannten Metalle in Frage, wie beispielsweise Titanyl TiO⁺⁺ und Chromyl CrO₂⁺⁺.

Das zum Kation A gehörige Anion X ist das einer anorganischen oder organischen Säure. Eine solche anorganische oder organische Säure kann einbasisch oder zweibasisch oder dreibasisch sein. Solche Säuren und ihre Anionen sind dem Fachmann bekannt. Beispiele für Anionen einbasischer anorganischer oder organischer Säuren sind: Fluorid, Bromid, Chlorid, Iodid, Nitrat, das Anion einer Alkancarbonsäure mit 1-18 C-Atomen und Benzoat; Beispiele für Anionen zweibasischer anorganischer oder organischer Säuren sind: Sulfat, Oxalat, Succinat, Fumarat, Maleinat, Phthalat und weitere; Beispiele für dreibasische anorganische oder organische Anionen sind: Phosphat oder Citrat. Bevorzugte Anionen X im Katalysator der Formel (III) sind: Fluorid, Chlorid, Bromid, lodid, Sulfat, Nitrat, Phosphat, Formiat, Acetat, Propionat, Oxalat, Butyrat, Citrat, Succinat, Fumarat, Maleinat, Benzoat, Phthalat, Decanoat, Stearat, Palmitat, und Laurinat. Besonders bevorzugte Anionen X sind: Chlorid, Bromid, lodid, Acetat, Laurinat, Stearat, Palmitat, Decanoat, Nitrat und Sulfat.

Als Kation B in den Katalysatoren der Formel (III) kommt eines aus der Gruppe der Alkali- oder Erdalkalikationen, der quaternären Ammonium-, Phosphonium-, Arsonium- oder Stibonium-Kationen und der ternären Sulfonium-Kationen in Frage.

Als (Erd)Alkalimetallkationen seien hierbei genannt: das Lithium-, Natrium-, Kalium-, Rubidium-, Cäsium-, Magnesium-, Calcium-, Strontium- und Bariumkation, bevorzugt die genannten Alkalimetallkationen, besonders bevorzugt das Natrium- und das Kaliumkation.

In bevorzugter Weise kommen als Kationen B solche der Formel (IV) 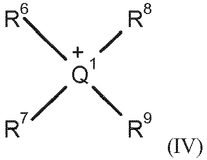 in Frage, worin
Q¹ für N, P, As oder Sb steht und
R⁶, R⁷, R⁸ und R⁹ unabhängig voneinander geradkettiges oder verzweigtes C₁-C₁₈ oder C₇-C₁₂-Aralkyl sind und einer der Reste R⁶-R⁹ auch C₆ - C₁₂ sein kann. In besonders bevorzugter Weise ist B ein Kation der Formel (V) 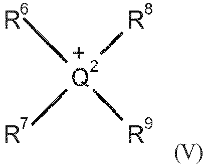 worin,
Q² für N oder P, bevorzugt für N steht.

In ganz besonders bevorzugter Weise treten im Rahmen der Formeln (IV) bzw. (V) an die Stelle der Reste R⁶, R⁷, R⁸ und R⁹ die Reste R¹⁶, R¹⁷, R¹⁸ bzw. R¹⁹, die unabhängig voneinander geradkettiges oder verzweigtes C₁-C₁₈-Alkyl oder C₇-C₈-Aralkyl bedeuten und einer der Reste R¹⁶ bis R¹⁹ auch Phenyl sein kann. In weiterhin ganz besonders bevorzugter Weise treten an die Stelle der Reste R¹⁶, R¹⁷, R¹⁸ bzw. R¹⁹, die Reste R²⁶, R²⁷, R²⁸ bzw. R²⁹, die unabhängig voneinander C₁-C₈-Alkyl oder Benzyl bedeuten und einer der Reste R²⁶ bis R²⁹ auch Phenyl sein kann.

Geradkettiges oder verzweigtes C₁-C₁₈-Alkyl ist beispielsweise Methyl, Ethyl, Propyl, Isopropyl, Butyl, Isobutyl, Hexyl, Octyl, Dodecyl, Hexadecyl oder Octadecyl. Bevorzugtes Alkyl hat 1-12 C-Atome, besonders bevorzugtes Alkyl hat 1-8 C-Atome.

C₇-C₁₂-Aralkyl ist beispielsweise Benzyl, Phenylethyl, Phenylpropyl, Naphthylmethyl oder Naphthylethyl; bevorzugtes Aralkyl ist Benzyl oder Phenylethyl, ganz besonders bevorzugtes Aralkyl ist Benzyl.

C₆-C₁₂-Aryl ist beispielsweise Phenyl, Naphthyl oder Biphenylyl, bevorzugt Phenyl.

Das Anion Y im Katalysator der Formel (III) ist ein Halogenidion, wie Fluorid, Chlorid, Bromid oder lodid, bevorzugt Bromid oder lodid, besonders bevorzugt lodid. Es kann jedoch auch die Bedeutung von anderen unter X genannten Anionen besitzen, wenn im konkreten Fall das Anion X Bromid oder lodid ist.

Der bifunktionelle Katalysator der Formel (III) wird in einer Menge von 0,005-5 Gew.-%, bevorzugt 0,01-3 Gew.-%, besonders bevorzugt 0,01-1 Gew.-%, bezogen auf das gesamte Umesterungsgemisch, eingesetzt.

Solche Katalysatoren können homogen gelöst auf den Kopf der Kolonne gegeben werden, wobei als Lösungsmittel Alkylencarbonat, Alkylenglykol, Alkohol oder Dialkylcarbonat, also systemeigene Lösungsmittel, zur Anwendung gelangen. Es ist selbstverständlich möglich, nicht lösliche Umesterungskatalysatoren einzusetzen, die auf den Zwischenböden oder inmitten der Füllkörper angeordnet sind. In einem solchen Fall kann die Dosierung eines gelösten Katalysators über (2) entfallen. Geeignete heterogene Katalysatoren sind beispielsweise:

lonentauscherharze mit funktionellen Gruppen aus tert. Aminen, quartären Ammoniumgruppen, wobei als Gegenionen Hydroxid, Chlorid oder Hydrogensulfat beispielsweise genannt seien, Sulfonsäuregruppen oder Carboxylgruppen, wobei für beide als Gegenionen Wasserstoff, Alkalimetalle oder Erdalkalimetalle beispielhaft genannt seien. Diese funktionellen Gruppen können entweder direkt oder über inerte Ketten an das Polymer gebunden sein (US 4,062,884 A, US 4,691,041 A, EP 298 167 A). Weiterhin genannt seien Alkali- oder Erdalkalisilikate, imprägniert auf Siliciumdioxidträgern, sowie Ammonium-ausgetauschte Zeolithe.

Das Herstellungsverfahren kann kontinuierlich oder diskontinuierlich durchgeführt werden. Bevorzugt ist eine kontinuierliche Fahrweise.

Im Verfahren werden die zyklische(n) Alkylencarbonatverbindung(en) und der oder die Alkylalkohol(e) bevorzugt im Molverhältnis von 1 : 0.1 bis 1 : 40, besonders bevorzugt von 1 : 1.0 bis 1 : 30, ganz besonders bevorzugt von 1 : 2.0 bis 1 : 20 eingesetzt. Dabei berücksichtigt das angegebene Molverhältnis nicht die Rückführung von zyklischer Alkylencarbonatverbindung oder Alkylalkohol in die Umesterungskolonne über einen oder mehrere Kopfkondensator(en) oder einen oder mehrere etwaig vorhandene(n) Sumpfverdampfer.

Der Katalysator wird bevorzugt zusammen mit dem Strom enthaltend das zyklische Alkylencarbonat in gelöster oder suspendierter Form in die Umesterungskolonne über eine Einführungsstelle, die oberhalb der Einführungsstellen des Alkylalkohols angeordnet ist, in die Kolonne eingebracht. Alternativ kann der Katalysator auch separat beispielsweise im Alkylalkohol, im Alkylenglykol oder in einem geeigneten inerten Lösungsmittel gelöst, zudosiert werden. Im Falle der Verwendung heterogener Katalysatoren können diese im Gemisch mit den genannten Füllkörpern, in geeigneter Form anstelle von Füllkörpern oder als Schüttung auf etwaig eingebaute Kolonnenböden eingesetzt werden.

Der Umsatz von Alkylencarbonat und Alkylalkohol zu Dialkylcarbonat und Alkylenglykol findet fast vollständig in einer Umesterungskolonne statt. In bevorzugten Ausführungsformen des Verfahrens zur Herstellung von Dialkylcarbonat kann der am Sumpf dieser Umesterungskolonne entnommene Flüssigkeitsstrom - gegebenenfalls nach Aufkonzentrierung - in einem oder mehreren weiteren Schritten einer weiteren Reaktion und/oder Reinigung unterzogen werden. Bevorzugt können einzelne oder alle solche weiteren Schritte in einer oder mehreren weiteren Kolonnen erfolgen.

Als Umesterungskolonne oder gegebenenfalls zweite bzw. weitere Kolonne(n) kommen dem Fachmann bekannte Kolonnen in Frage. Dies sind beispielsweise Destillations- bzw. Rektifikationskolonnen, bevorzugt Reaktivdestillations- bzw. Reaktivrektifikationskolonnen.

Ein geeignetes Kolonnendesign der im Verfahren eingesetzten Destillations- und/oder Reaktionskolonnen, welches sowohl die Festlegung der Kolonnenhöhe, des Kolonnendurchmessers, die Auswahl der Kolonneneinbauten als auch die Dimensionierung der Zulauf- und Entnahmeleitungen umfasst, ist dem Fachmann bekannt und kann der einschlägigen Literatur (z.B. Distillation Design, Henry Z. Kister, Mc Graw Hill; Distillation Operation, Henry Z. Kister, Mc Graw Hill; Perry's Chemical Engineering Handbook; Perry & Green) entnommen werden.

Hinsichtlich der Kondensation im Kopfkondensator sind unterschiedliche Ausführungsformen denkbar. Als Kopfkondensatoren eignen sich beispielsweise Rohrbündel- oder Plattenwärmetauscher. Das Verhältnis d₁/D₁ von Durchmesser der Dampfleitung von der Kolonne zum Kondensator (d₁) zu Kolonnendurchmesser der Destillationskolonne (D₁) liegt bevorzugt im Bereich von 0,2 bis 1,0, besonders bevorzugt im Bereich von 0,5 bis 1. In einer besonders bevorzugten Ausführungsform kann der Kopfkondensator in die Destillationskolonne integriert sein, so dass zwischen Destillationskolonne und Kopfkondensator keine Dampfleitung erforderlich ist. Das Verhältnis d₁/D₁ beträgt in diesem Fall 1. Dabei kann der Kolonnenquerschnitt nach Eintritt in den Kopfkondensator unter Umständen auch dem Kondensationsfortschritt angepasst werden.

Bei einigen Kondensatorformen kann es von Vorteil sein, den Kolonnenquerschnitt variabel zu gestalten. Ist die Führung der zu kondensierenden Dämpfe beispielsweise von unten nach oben, so nimmt die Dampfmenge nach oben hin ab. Durch eine Reduktion des Kolonnendurchmessers in Richtung Kolonnenkopf, wird der für den Dampf verfügbare Kolonnenquerschnitt der nach oben hin abnehmenden Dampfmenge angepasst. Dabei muss die Entnahme der nicht kondensierten Dämpfe nicht zwangsläufig oben erfolgen. Wird beispielsweise eine Konstruktion gewählt, bei der ein Platten- oder Rohrbündel von oben in die Kolonne einfügt wird, so kann sich die Entnahme der nicht kondensierten Dämpfe auch seitlich befinden.

Die Umesterungskolonne enthält bevorzugt wenigstens einen Verstärkungsteil im oberen Teil der Kolonne und wenigstens eine Reaktionszone unterhalb des Verstärkungsteils. Der Verstärkungsteil weist unabhängig 0 bis 30, bevorzugt 0,1 bis 30 theoretische Stufen auf.

In bevorzugten Ausführungsformen weist die Umesterungskolonne unterhalb einer Reaktionszone wenigstens einen Abtriebsteil auf, der über 0 bis 20, bevorzugt 1 bis 10 theoretische Stufen verfügt.

Die Umesterungskolonne kann weiterhin bevorzugt mit einem oder mehreren Sumpfverdampfer(n) ausgestattet sein. Bei Ausführung der Umesterungskolonne mit einem Abtriebsteil wird bevorzugt zusätzlich ein Sumpfverdampfer eingesetzt, welcher die aus dem Abtriebsteil ablaufende Flüssigkeit ganz oder teilweise verdampft. Dieser ganz oder teilweise verdampfte Flüssigkeitsstrom wird ganz oder teilweise wieder in die Umesterungskolonne zurückgeführt. Im Falle einer Ausführungsform ohne Abtriebsteil wird in einem gegebenenfalls eingesetzten Sumpfverdampfer die aus der Reaktionszone ablaufende Flüssigkeit ganz oder teilweise verdampft und ganz oder teilweise wieder in die Umesterungskolonne zurückgeführt.

Der oder die Verstärkungsteil(e) können in bevorzugten Ausführungsformen zusammen mit dem oder den Reaktionsteil(en) und gegebenenfalls wenigstens einem Abtriebsteil in der Umesterungskolonne untergebracht werden. Dabei wird das aus der oder den Reaktionszone(n) kommende dampfförmige Gemisch von unten in eine untere Sektion des Verstärkungsteils bzw. gegebenenfalls den unteren Verstärkungsteil geleitet, wobei eine Abreicherung des Alkylencarbonats bzw. Alkylenglykols stattfindet.

Unterhalb der Reaktionszone und eines gegebenenfalls vorhandenen Abtriebsteils wird ein Gemisch enthaltend Alkylenglykol, überschüssigem oder nicht umgesetztem Alkylencarbonat, Alkylalkohol, Dialkylcarbonat, Umesterungskatalysatoren und bei der Reaktion entstehende oder bereits in den Edukten enthaltene schwersiedende Verbindungen erhalten. Bei Verwendung eines Abtriebsteils wird der Gehalt an leichtsiedenden Verbindungen, wie beispielsweise Dialkylcarbonat und Alkohol reduziert, wobei in Anwesenheit des Umesterungskatalysators unter Umständen weiteres Dialkylcarbonat und Alkylenglykol gebildet werden. Die hierzu erforderliche Energie, wird bevorzugt durch einen oder mehrere Verdampfer zugeführt.

In allen Abschnitten der Umesterungskolonne, d.h. sowohl in Verstärkungs- und gegebenenfalls Abtriebsteil als auch in der Reaktionszone können Füllkörper oder geordnete Packungen zum Einsatz kommen. Die zu verwendenden Füllkörper bzw. geordneten Packungen sind die für Destillationen üblichen, wie sie beispielsweise in Ullmann's Encyclopädie der Technischen Chemie, 4. Aufl,, Bd. 2, S. 528 ff. beschrieben sind. Als Beispiele für Füllkörper seien Raschig- oder Pall- und Novaloxringe, Berl-, Intalex- oder Torussättel, Interpackkörper und als Beispiele für geordnete Packungen seien Blech und Gewebepackungen (wie z.B. BX-Packungen, Montz Pak, Mellapak, Melladur, Kerapak und CY-Packung) aus verschiedenen Materialien, wie Glas, Steinzeug, Porzellan, Edelstahl, Kunststoff genannt. Bevorzugt sind Füllkörper und geordnete Packungen, die eine große Oberfläche, eine gute Benetzung sowie ausreichende Verweilzeit der Flüssigphase aufweisen. Diese sind beispielsweise Pall- und Novolaxringe, Berlsättel, BX-Packungen, Montz Pak, Mellapak, Melladur, Kerapak und CY-Packungen.

Alternativ eignen sich auch Kolonnenböden, wie beispielsweise Sieb-, Glocken-, Ventil-, Tunnelböden. In der oder den Reaktionszone(n) der Umesterungskolonne sind Kolonnenböden mit hohen Verweilzeiten bei gutem Stoffaustausch, beispielsweise Glockenböden, Ventil- oder Tunnelböden mit hohen Überlaufwehren, besonders bevorzugt. Die theoretische Bodenzahl der Reaktionszone beträgt vorzugsweise 3 bis 50, besonders bevorzugt 10 bis 50 und ganz besonders bevorzugt 10 bis 40. Der Flüssigkeits-Hold-up beträgt vorzugsweise 1 bis 80%, besonders bevorzugt 5 bis 70% und ganz besonders bevorzugt 7 bis 60% des Kolonneninnenvolumens der Reaktionszone. Die genauere Auslegung der Reaktionszone(n), des gegebenenfalls zu verwendendes Abtriebsteils und des oder der Verstärkerteil(e) kann vom Fachmann vorgenommen werden.

Die Temperatur der Reaktionszone(n) liegt bevorzugt im Bereich von 20 bis 200°C, besonders bevorzugt von 40 bis 180°C, ganz besonders bevorzugt von 50 bis 160°C. Es ist von Vorteil, die Umesterung nicht nur bei Normaldruck, sondern auch bei erhöhtem oder erniedrigtem Druck durchzuführen. Der Druck der Reaktionszone liegt daher bevorzugt im Bereich von 0,2 bis 20 bar, besonders bevorzugt von 0,3 bis 10 bar, ganz besonders bevorzugt von 0,4 bis 5 bar. Bei den vorangehend und im Folgenden aufgeführten Druckangaben handelt es sich - sofern nichts anderes explizit erwähnt - um absolute Druckangaben.

Bevorzugt wird das am Kopf der Umesterungskolonne in dem Verfahren zur Herstellung des Dialkylcarbonates entnommene Dampfgemisch enthaltend Dialkylcarbonat und Alkylalkohol nach Kondensation am Kopf der Umesterungskolonne ganz oder teilweise wenigstens einem weiteren Verfahrensschritt enthaltend wenigstens eine Destillationskolonne zur Trennung von Dialkylcarbonat und Alkylalkohol zugeführt.

Die Trennung des Dialkylcarbonats und des Alkylalkohols erfolgt bevorzugt destillativ in einer oder mehreren Destillationskolonnen oder in einer Kombination aus Destillation und Membrantrennung - im Folgenden als Hybridprozess - bezeichnet (siehe z. B. US-4,162,200 A, EP 581 115 B1, EP 592 883 B1 und WO 2007/096343A1).

Bilden Alkylalkohol und Dialkylcarbonat ein Azeotrop (z.B. Methanol und Dimethylcarbonat) so kann auch ein zweistufiges Verfahren wie beispielsweise ein Zweidruckverfahren, eine Extraktivdestillation, eine Heteroazeotropdestillation mit einem niedrigsiedenden Schleppmittel oder ein Hybridverfahren verwendet werden. Besonders bevorzugt wird das Zweidruckverfahren oder ein Hybridverfahren angewendet.

Ganz besonders bevorzugt wird die Trennung des Dialkylcarbonats und des Alkylalkohols - selbst in dem Falle, dass das Dialkylcarbonat und der Alkylalkohol ein Azeotrop bilden - in einer einzelnen Destillationskolonne durchgeführt. Diese Destillationskolonne wird bei einem Druck betrieben, der höher ist als der Druck der Umesterungskolonne(n). Der Betriebsdruck der Destillationskolonne liegt im Bereich von 1 bis 50 bar, vorzugsweise zwischen 2 und 20 bar Am Sumpf der Destillationskolonne wird das nahezu reine Dialkylcarbonat entnommen und am Kopf ein Gemisch aus Dialkylcarbonat und Alkylalkohol. Dieses Gemisch wird der oder den Umesterungskolonne(n) ganz oder teilweise zugeführt. Wird das Verfahren zur Herstellung von Dialkylcarbonat mit einem Verfahren zur Herstellung von Diarylcarbonat, welches durch Umesterung dieses Dialkylcarbonates mit einer aromatischen Hydroxyverbindung gebildet wird, gekoppelt, so kann ein Teil des Gemisches aus Dialkylcarbonat und Alkylalkohol, welches am Kopf der Destillationskolonne entnommen wird, einem entsprechenden Aufarbeitungsschritt für Alkylalkohol und Dialkylcarbonat in der Verfahrensstufe zur Herstellung von Diarylcarbonat zugeführt werden.

In einer besonders bevorzugten Ausführung, wenn das Dialkylcarbonat und der Alkylalkohol ein Azeotrop bilden, ist dieser Aufarbeitungsschritt ein Zweidruckverfahren. Solche Verfahren sind dem Fachmann grundsätzlich bekannt (vgl. z.B. Ullmann's Encyclopedia of Industrial Chemistry, Vol. 7, 2007, Kap. 6.4. und 6.5.; Chemie Ingenieur Technik (67) 11 / 95).

Bilden Alkylalkohol und Dialkylcarbonat ein Azeotrop, so weist das Destillat einer ersten Destillationskolonne des Verfahrensschritts zur Trennung von Dialkylcarbonat und Alkylalkohol vorzugsweise nahezu azeotrope Zusammensetzung auf. In diesem Fall wird dieses bevorzugt in einem Zweidruckverfahren wenigstens einer weiteren Destillationskolonne zugeführt, die bei einem Betriebsdruck arbeitet, der unter dem der ersten Destillationskolonne liegt. Durch den unterschiedlichen Betriebsdruck verschiebt sich die Lage des Azeotrops hin zu geringeren Anteilen an Alkylalkohol. Man erhält als Sumpfprodukt dieser zweiten oder weiteren Destillationskolonne(n) Alkylalkohol in einer Reinheit von 90 bis 100 Gew.-%, bezogen auf das Gesamtgewicht des isolierten Sumpfproduktes, und als Destillat ein nahezu azeotropes Gemisch. Die bei geringerem Betriebsdruck arbeitende zweite oder weitere(n) Destillationskolonne(n) wird in ganz besonders bevorzugten Ausführungsformen vorzugsweise mit der Kondensationswärme des oder der Kopfkondensator(en) der ersten Destillationskolonne betrieben.

Beim Zweidruckverfahren macht man sich die Druckabhängigkeit der azeotropen Zusammensetzung eines Zweistoffgemisches zunutze. Bei einem Gemisch aus Alkylalkohol und Dialkylcarbonat, wie beispielsweise Methanol und Dimethylcarbonat, verschiebt sich die azeotrope Zusammensetzung mit zunehmendem Druck zu höheren Alkylalkoholgehalten. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechende azeotropen Zusammensetzung liegt, so erhält man als Destillat ein Gemisch mit nahezu azeotroper Zusammensetzung und als Sumpfprodukt nahezu reines Dialkylcarbonat. Das so erhaltene azeotrope Gemisch wird einer weiteren Destillationskolonne (Alkylalkoholkolonne) zugeführt. Diese arbeitet bei einem im Vergleich zur Dialkylcarbonatkolonne geringeren Betriebsdruck. Dadurch verschiebt sich die Lage des Azeotrops hin zu geringeren Alkylalkoholgehalten. Hierdurch ist es möglich, das in der Dialkylcarbonatkolonne erhaltene azeotrope Gemisch in ein Destillat mit nahezu azeotroper Zusammensetzung und nahezu reinen Alkylalkohol zu trennen. Das Destillat der Alkylalkoholkolonne wird wieder der Dialkylcarbonatkolonne an geeigneter Stelle zugeführt.

Der Betriebsdruck der Alkylalkoholkolonne wird vorzugsweise so gewählt, dass man diese mit der Abwärme der Dialkylcarbonatkolonne betreiben kann. Der Betriebsdruck liegt dabei zwischen 0,1 und 1 bar vorzugsweise zwischen 0,3 und 1 bar. Der Betriebsdruck der Dialkylcarbonatkolonne liegt im Bereich von 1 bis 50 bar, vorzugsweise zwischen 2 und 20 bar.

Ein weiteres bevorzugtes Verfahren zur Trennung von Azeotropen aus Alkylalkohol und Dialkylcarbonat ist das Hybridverfahren. Beim Hybridverfahren erfolgt die Trennung eines Zweistoffgemisches mittels einer Kombination aus Destillation und Membranverfahren. Dabei macht man sich die Tatsache zunutze, dass man die Komponenten aufgrund ihrer polaren Eigenschaften und ihres unterschiedlichen Molekulargewichts mittels Membranen zumindest teilweise voneinander trennen kann. Im Falle eines Gemisches aus Alkylalkohol und Dialkylcarbonat, wie beispielsweise Methanol und Dimethylcarbonat, erhält man bei Verwendung geeigneter Membranen mittels Pervaporation oder Dampfpermeation ein Alkylalkohol-reiches Gemisch als Permeat und ein an Alkylalkohol verarmtes Gemisch als Retentat. Führt man ein Gemisch dieser beiden Komponenten einer Kolonne (Dialkylcarbonatkolonne) zu, wobei der Alkylalkoholgehalt unterhalb der für den Betriebsdruck dieser Kolonne entsprechenden azeotropen Zusammensetzung liegt, so erhält man als Destillat ein Gemisch mit im Vergleich zum Zulauf deutlich erhöhtem Alkylalkoholgehalt und als Sumpfprodukt nahezu reines Dialkylcarbonat.

Im Falle eines Hybridverfahrens aus Destillation und Dampfpermeation wird das Destillat der Kolonne dampfförmig entnommen. Das so erhaltene dampfförmige Gemisch wird, gegebenenfalls nach Überhitzung, einer Dampfpermeation zugeführt. Diese wird so betrieben, dass man auf der Retentatseite nahezu den Betriebsdruck der Kolonne und auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Alkylalkohol-reiche Fraktion mit einem Alkylalkoholgehalt von mindestens 70 Gew.-%, bevorzugt mindestens 90 Gew.-%, bezogen auf das Gesamtgewicht der Fraktion. Das Retentat, welches einen im Vergleich zum Destillat der Kolonne einen reduzierten Alkylalkoholanteil enthält, wird gegebenenfalls kondensiert und wieder der Destillationskolonne zugeführt.

Im Falle eines Hybridverfahrens aus Destillation und Pervaporation wird das Destillat der Kolonne flüssig entnommen. Das so erhaltene Gemisch wird gegebenenfalls nach Erhitzung einer Pervaporation zugeführt. Diese wird so betrieben, dass man auf der Retentatseite einen im Vergleich zur Kolonne identischen oder erhöhten Betriebsdruck auf der Permeatseite einen geringeren Druck einstellt. Der Betriebsdruck der Kolonne liegt dabei im Bereich von 1 bis 50 bar, bevorzugt zwischen 1 und 20 und besonders bevorzugt zwischen 2 und 10 bar. Der Druck auf der Permeatseite liegt zwischen 0,05 und 2 bar. Dabei erhält man auf der Permeatseite eine Alkylalkohol-reiche dampfförmige Fraktion mit einem Alkylalkoholgehalt von mindestens 70 Gew.-%, bevorzugt mindestens 90 Gew.-%, bezogen auf das Gesamtgewicht der Fraktion. Das flüssige Retentat, welches einen im Vergleich zum Destillat der Kolonne reduzierten Alkylalkoholanteil enthält, wird wieder der Destillationskolonne zugeführt. Durch die Verdampfung des Permeats wird Wärme benötigt, die gegebenenfalls nicht in ausreichendem Maße im Zulaufstrom zur Pervaporation enthalten ist. Daher kann eine Membrantrennung mittels Pervarporation gegebenenfalls mit zusätzlichen Wärmetauschern beheizt werden, wobei diese integriert oder gegebenenfalls zwischen mehreren hintereinander geschalteten Pervaporationsschritten angebracht sind.

Die Trennung von Dialkylcarbonat und Alkylalkohol erfolgt im Falle eines Hybridverfahrens besonders bevorzugt mittels einer Kombination aus Destillation und Dampfpermeation.

Die zur Trennung von Alkylalkohol und Dialkylcarbonat erforderliche Wärme wird bei einer Temperatur zwischen 100°C und 300°C, vorzugsweise zwischen 100°C und 230°C, und besonders bevorzugt zwischen 120°C und 200°C zugeführt.

Die Destillationskolonne(n) zur Aufreinigung des Dialkylcarbonats verfügen bevorzugt über einen Verstärkungsteil mit bevorzugt 5 bis 40 theoretischen Stufen zur Aufkonzentrierung des Alkylalkohols und einen Abtriebsteil mit bevorzugt 5 bis 40 theoretischen Stufen zur Aufkonzentrierung des Dialkylcarbonates.

Zur Aufarbeitung wird das Sumpfprodukt der Umesterungskolonne weiteren Prozessschritten zugeführt. Hierzu können unterschiedliche Wege beschritten werden wie z.B. a) weitere Reaktionen durchführen, um unerwünschte Nebenkomponenten zu entfernen (z. B. EP 889 025) bzw. b) durch eine Reaktion in Verbindungen zu überführen, die leichter abtrennbar sind, (z. B. EP 1174 406) oder c) Trennoperationen vornehmen wie z.B. Destillation, Extraktion etc. (z. B. EP 569 812).

Nach diesen Verfahrensschritten wird das vorgereinigte Sumpfprodukt der Umesterungskolonne, das nun Alkylenglykol in einer Konzentration von größer 95 Gew. %, bevorzugt größer 97 Gew. % und besonders bevorzugt größer 98 Gew.% enthält, einer Destillationskolonne zugeführt, die diesen Produktstrom noch weiter aufreinigt. Diese Destillationskolonne wird in den Beispielen auch als Alkylenglykolaufarbeitungskolonne bezeichnet.

Wahlweise kann der evtl. noch vorhandene homogene Katalysator vor der Einspeisung in die Destillationskolonne beispielsweise durch einen Fallfilmverdampfer (EP 569 812 A) oder in der Kolonne selbst mit dem dort anfallenden Sumpfprodukt ausgeschleust werden.

Die Destillationskolonne enthält bevorzugt wenigstens einen Verstärkungsteil im oberen Teil der Kolonne und wenigstens einen Abtriebsteil unterhalb des Verstärkungsteils. Die Kolonne weist 0 bis 100, bevorzugt 0,1 bis 100 theoretische Stufen auf. Wahlweise kann das Alkylenglykol am Kopf oder in einem Seitenabzug der Kolone entnommen werden, vorzugsweise wird es als Seitenstrom entnommen.

Die Kolonne wird bei 1 bis 2000 mbar, bevorzugt bei 10 bis 1000 mbar betrieben.

Das Verfahren zur Herstellung von Dialkylcarbonat wird bevorzugt kontinuierlich durchgeführt.

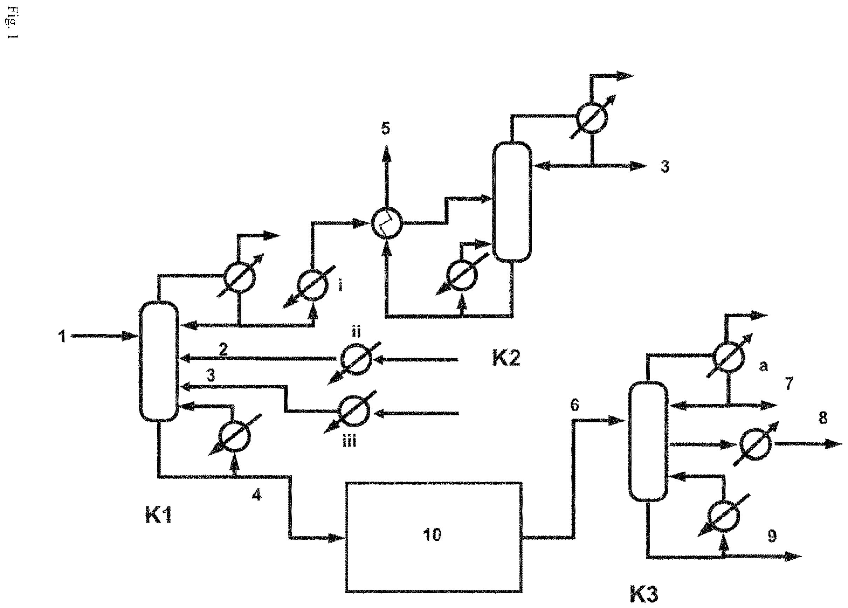

### Erläuterungen zur Figur 1:

- K1: Umesterungskolonne
- K2: Destillationskolonne zur Trennung des Gemisches enthaltend Dialkylcarbonat und Alkylalkohol
- K3: Destillationskolonne zur Aufreinigung des Alkylenglykols

- 1: Eduktstrom enthaltend Alkylencarbonat und/oder optional Katalysator
- 2: Eduktstrom enthaltend Alkylalkohol und Dialkylcarbonat
- 3: Eduktstrom enthaltend nahezu reinen Alkylalkohol
- 4: Strom enthaltend Alkylenglykol
- 5: Strom enthaltend aufgereinigtes Dialkylcarbonat
- 6: Strom enthaltend vorgereinigtes Alkylenglykol
- 7: Strom enthaltend u.a. leichtsiedende Komponenten
- 8: Produktstrom enthaltend aufgereinigtes Alkylenglykol
- 9: Strom enthaltend u.a. hochsiedende Rückstände
- 10: Prozessschritte zur Vorreinigung des Alkylenglykols
- a: Wärmetauscher zur Rückgewinnung der Kondensationsenergie
- i, ii, iii: Wärmetauscher, in denen die rückgewonnene Kondensationsenergie zur Erwärmung von Produktströmen eingesetzt werden kann

**Fig. 1** beschreibt einen Umesterungsschritt von Alkylencarbonat und Alkylalkohol mittels Reaktivrektifikation in einer ersten Umesterungskolonne (K1) im Allgemeinen und die Aufarbeitung des am Kopf der Umesterungskolonne anfallenden Gemisches enthaltend Dialkylcarbonat und Alkylalkohol in einer Destillationskolonne (K2) und die abschließende Destillation des Alkylenglykols in einer weiteren Destillationskolonne (K3).

### Beispiele

Anhand eines Beispiels wird nun die bevorzugte Betriebsweise für das erfindungsgemäße Verfahren detailliert aufgezeigt. Beispiel 1 und 2 zeigen die bevorzugte Art der Abwärmenutzung. Dieses Beispiel soll auf keine Weise als Limitierung der Erfindung ausgelegt werden. Der Vorteil dieser Erfindung, nämlich die Verringerung des Verbrauchs an frischem Heizdampf, gegenüber anderen Betriebsarten wird im Vergleichsbeispiel dargestellt.

### Beispiel 1:

Eine reaktive Destillationskolonne bestehend aus einem Verstärkungsteil mit 9 theoretischen Stufen, einer Reaktionszone mit 25 Reaktionsböden (Holdup/Boden: 0,6 m³) und einem Abtriebsteil mit 4 theoretischen Stufen wird bei einem am Kopf der Kolonne gemessenen Druck von 400 mbar (absolut) und einem Rücklaufverhältnis von 0,5 betrieben.

In den oberen Kolonnenbereich direkt oberhalb des ersten Reaktionsbodens werden kontinuierlich 9000 kg/h Ethylencarbonat und 175 kg/h eines Gemisches mit 33,3 Gew.-% KOH und 66,7 Gew.-% Ethylenglykol zudosiert. Zwischen dem 8. und 9. Reaktionsboden werden 16854 kg/h eines Dampfgemisches mit 83,7 Gew.-% Methanol und 16,3 Gew.-% Dimethylcarbonat zugeführt. Das Methanol-Dimethylcarbonat-Gemisch wird bei 61°C verdampft und überhitzt. Zusätzlich dazu werden am unteren Ende der Reaktionszone 7078 kg/h eines Dampfgemisches mit >99,9 Gew.-% Methanol zugeführt. Beide Methanolströme werden bei 62°C verdampft und überhitzt. Dazu sind 443 t/h heißes Kreislaufwasser bei 90°C und nur 1,1 t/h Heizdampf bei 1,5 bar (0,7 MW) nötig.

Als Kopfprodukt erhält man 26077 kg/h Destillat mit einer Zusammensetzung von 54,1 Gew.-% Methanol und 45,6 Gew.-% Dimethylcarbonat. Das Sumpfprodukt (7229 kg/h) beinhaltet unter anderem 88,9 Gew.-% Ethylenglykol, 6,7 Gew.-% Methanol und 2,5 Gew.-% Ethylencarbonat. Sowohl Kopf- als auch Sumpfprodukt werden noch weiteren Aufreinigungsschritten zugeführt.

Die Ethylenglykol-Aufarbeitungskolonne besteht aus 58 theoretischen Stufen. Die Kolonne wird bei einem am Kopf der Kolonne gemessenen Druck von 50 mbar (absolut) und einem Rücklaufverhältnis von 36 betrieben. 7026 kg/h rohes Ethylenglykol mit einem Ethylenglykolgehalt von ca. 99 Gew.-% wird der Kolonne zwischen der 49. und 50. theoretischen Stufe zugeführt. Zusätzlich dazu wird 902 kg/h rohes Ethylenglykol aus der Katalysator-Rückgewinnungskolonne zwischen der 8. und 9. theoretischen Stufe zurückgeführt.

Als Kopfprodukt erhält man 492 kg/h Destillat bei 105°C mit einer Zusammensetzung von 97,6 Gew.-% Ethylenglykol und 0,4 Gew.-% Ethylencarbonat, das später als Betriebsflüssigkeit für das Vakuumsystem eingesetzt werden kann. Dabei wird der Kopfkondensator zur Erzeugung von 443 t/h heißem Kreislaufwasser bei 90°C genutzt. Das Sumpfprodukt (1200 kg/h) beinhaltet unter anderem 94,3 Gew.-% Ethylenglykol und 5,0 Gew.-% KOH, das weiteren Aufarbeitungsschritten zur Rückgewinnung des Katalysators zugeführt wird. 6230 kg/h Ethylenglykol (>99,9 Gew.-%) wird als Seitenprodukt zwischen der 28. und 29. theoretischen Stufe gewonnen.

### Beispiel 2:

Eine reaktive Destillationskolonne, bestehend aus einem Verstärkungsteil mit 9 theoretischen Stufen, einer Reaktionszone mit 25 Reaktionsböden (Holdup/Boden: 0,6 m³) und einem Abtriebsteil mit 4 theoretischen Stufen wird bei einem am Kopf der Kolonne gemessenen Druck von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 175 kg/h eines Gemisches mit 33,3 Gew.-% KOH und 66,7 Gew.-% Ethylenglykol zudosiert. Zwischen dem 8. und 9. Reaktionsboden werden 21371 kg/h eines Dampfgemisches mit 83,7 Gew.-% Methanol und 16,3 Gew.-% Dimethylcarbonat zugeführt. Das Methanol-Dimethylcarbonat-Gemisch wird bei 61°C verdampft und überhitzt. Als Heizmittel wird 373 t/h heißes Kreislaufwasser bei 90°C eingesetzt. Zusätzlich dazu werden am unteren Ende der Reaktionszone 7123 kg/h eines Dampfgemisches mit 99,5 Gew.-% Methanol und 0,41 Gew.-% Ethylenglykol zugeführt. Dieses fast reine Methanol wird bei 74°C verdampft und überhitzt. Dazu werden 3,9 t/h Heizdampf bei 1,5 bar (2,4 MW) benötigt.

Als Kopfprodukt erhält man 30644 kg/h Destillat mit einer Zusammensetzung von 59 Gew.-% Methanol und 41 Gew.-% Dimethylcarbonat. Das Sumpfprodukt (7019 kg/h) beinhaltet unter anderem 92,3 Gew.-% Ethylenglykol, 6,7 Gew.-% Methanol und 477 ppm Ethylencarbonat. Sowohl Kopf- als auch Sumpfprodukt werden noch weiteren Aufreinigungsschritten zugeführt.

Die Ethylenglykolaufarbeitungskolonne besteht aus 58 theoretischen Stufen. Die Kolonne wird bei einem am Kopf der Kolonne gemessenen Druck von 50 mbar (absolut) und einem Rücklaufverhältnis von 36 betrieben. 6876 kg/h rohes Ethylenglykol mit einem Ethylenglykolgehalt von ca. 99 Gew.-% wird der Kolonne zwischen der 49. und 50. theoretischen Stufe zugeführt. Zusätzlich dazu wird 909 kg/h rohes Ethylenglykol aus der Katalysatorrückgewinnungskolonne zwischen der 8. und 9. theoretischen Stufe zurückgeführt.

Als Kopfprodukt erhält man 500 kg/h Destillat bei 105°C mit einer Zusammensetzung von 99,4 Gew.-% Ethylenglykol und 0,4 Gew.-% Ethylencarbonat, das später als Betriebsflüssigkeit für das Vakuumsystem eingesetzt werden kann. Dabei wird der Kopfkondensator zur Erzeugung von 441 t/h heißem Kreislaufwasser bei 90°C genutzt. Das Sumpfprodukt (1200 kg/h) beinhaltet unter anderem 94,8 Gew.-% Ethylenglykol und 4,9 Gew.-% KOH, das weiteren Aufarbeitungsschritten zur Rückgewinnung des Katalysators zugeführt wird. 6203 kg/h Ethylenglykol (>99,9 Gew.-%) wird als Seitenprodukt zwischen der 28. und 29. theoretischen Stufe gewonnen.

### Vergleichsbeispiel 1

Eine reaktive Destillationskolonne, bestehend aus einem Verstärkungsteil mit 9 theoretischen Stufen, einer Reaktionszone mit 25 Reaktionsböden (Holdup/Boden: 0,6 m³) und einem Abtriebsteil mit 4 theoretischen Stufen wird bei einem am Kopf der Kolonne gemessenen Druck von 400 mbar (absolut) und einem Rücklaufverhältnis von 0,66 betrieben.

In den oberen Kolonnenbereich direkt oberhalb des ersten Reaktionsbodens werden kontinuierlich 9000 kg/h Ethylencarbonat und 175 kg/h eines Gemisches mit 33,3 Gew.-% KOH und 66,7 Gew.-% Ethylenglykol zudosiert. Zwischen dem 8. und 9. Reaktionsboden werden 21371 kg/h eines Dampfgemisches mit 83,7 Gew.-% Methanol und 16,3 Gew.-% Dimethylcarbonat zugeführt. Zusätzlich dazu werden am unteren Ende der Reaktionszone 7123 kg/h eines Dampfgemisches mit 99,5 Gew.-% Methanol und 0,41 Gew.-% Ethylenglykol zugeführt. Beide Methanolströme werden bei 61°C bzw. 74°C verdampft und überhitzt. Dazu werden 10,8 t/h Heizdampf bei 1,5 bar (6,8 MW) benötigt.

Als Kopfprodukt erhält man 30644 kg/h Destillat mit einer Zusammensetzung von 59 Gew.-% Methanol und 41 Gew.-% Dimethylcarbonat. Das Sumpfprodukt (7019 kg/h) beinhaltet unter anderem 92,3 Gew.-% Ethylenglykol, 6,7 Gew.-% Methanol und 477 ppm Ethylencarbonat. Sowohl Kopf- als auch Sumpfprodukt werden noch weiteren Aufreinigungsschritten zugeführt.

## Patentansprüche

1. Verfahren zur kontinuierlichen Herstellung sowohl von Dialkylcarbonat der Formel
(R¹O)₂CO,
in der R¹ für ein geradkettiges oder verzweigtes C1-C4-Alkyl steht,
als auch von Alkylenglykol der Formel
HO-R²-OH
in der R² ein C₂ - C₄-Alkylen darstellt
durch Umesterung von zyklischem Alkylencarbonat mit einem Alkylalkohol der Formel
R¹OH
in der R¹ die obige Bedeutung hat,
in Gegenwart eines Katalysators, **dadurch gekennzeichnet, dass** die Kondensationswärme der Alkylenglykol-Aufarbeitungskolonne zurückgewonnen und direkt oder indirekt zur Verdampfung der der Umesterungskolonne zugeführten Ströme verwendet wird, die Alkylalkohol enthalten.

2. Verfahren zur Herstellung von Dialkylcarbonat nach Anspruch 1, **dadurch gekennzeichnet, dass** die Kondensationswärme aus der Alkylenglykol-Aufarbeitungskolonne zur Vorerhitzung des Feeds in der Dialkylcarbonat-Aufarbeitungskolonne oder zur Verdampfung des Alkylalkohols für die Umesterungskolonne eingesetzt wird. Besonders bevorzugt wird diese Wärme zur Verdampfung des reinen Alkylalkohols und/oder des Dialkylcarbonat enthaltenden Alkylalkohols für die Umesterungskolonne eingesetzt.

3. Verfahren nach Anspruch 1 oder 2, **dadurch gekennzeichnet, dass** die Umesterungskolonne einen Abtriebsteil enthält, der unterhalb der Reaktionszone angeordnet ist.

4. Verfahren zur Herstellung von Dialkylcarbonat nach einem der Ansprüche 1 bis 3, **dadurch gekennzeichnet, dass** die Umesterung in wenigstens einer Umesterungskolonne in Gegenwart eines Katalysators im Gegenstrom so geführt wird, dass Alkylencarbonat in den oberen Teil der Kolonne und ein Dialkylcarbonat enthaltender Alkylalkohol, dessen Dialkylcarbonatgehalt bei 0,2 bis 30 Gew.-% liegt, in den mittleren oder unteren Teil der Reaktionszone der wenigstens einen Umesterungskolonne eingeführt wird.

5. Verfahren zur Herstellung von Dialkylcarbonat nach Anspruch 4, **dadurch gekennzeichnet, dass** der Umesterungskolonne ein weiterer Strom enthaltend nahezu reinen Alkylalkohol zugeführt wird, dessen Einführstelle unterhalb der Einführstelle des Dialkylcarbonat enthaltenden Alkylalkoholstromes angeordnet ist.

6. Verfahren zur Herstellung von Dialkylcarbonat nach einem der Ansprüche 1 bis 5, **dadurch gekennzeichnet, dass** als zyklisches Alkylencarbonat Ethylen- oder Propylencarbonat und als Alkylalkohol Methanol oder Ethanol verwendet wird.

7. Verfahren zur Herstellung von Dialkylcarbonat nach einem der Ansprüche 1 bis 6, **dadurch gekennzeichnet, dass** Temperatur der Reaktionszone(n) im Bereich von 20 bis 200°C liegt und der Druck im Bereich von 0,2 bis 20 bar liegt.

## Claims

1. Process for continuously preparing both dialkyl carbonate of the formula
(R¹O)₂CO,
in which R¹ is a straight-chain or branched C1-C4-alkyl
and alkylene glycol of the formula
HO-R²-OH
in which R² is a C₂-C₄-alkylene
by transesterifying cyclic alkylene carbonate with an alkyl alcohol of the formula
R¹OH
in which R¹ is as defined above in the presence of a catalyst, **characterized in that** the heat of condensation of the alkylene glycol workup column is recovered and used directly or indirectly to vaporize the streams containing alkyl alcohol which are fed to the transesterification column.

2. Process for preparing dialkyl carbonate according to Claim 1, **characterized in that** the heat of condensation from the alkylene glycol workup column is used to preheat the feed in the dialkyl carbonate workup column or to vaporize the alkyl alcohol for the transesterification column. Particular preference is given to using this heat to vaporize the pure alkyl alcohol and/or the dialkyl carbonate-containing alkyl alcohol for the transesterification column.

3. Process according to Claim 1 or 2, **characterized in that** the transesterification column has a stripping section beneath the reaction zone.

4. Process for preparing dialkyl carbonate according to any of Claims 1 to 3, **characterized in that** the transesterification is conducted in countercurrent in the presence of a catalyst in at least one transesterification column in such a way that alkylene carbonate is introduced into the upper section of the column and a dialkyl carbonate-containing alkyl alcohol having a dialkyl carbonate content of 0.2% to 30% by weight into the middle or lower section of the reaction zone of the at least one transesterification column.

5. Process for preparing dialkyl carbonate according to Claim 4, **characterized in that** the transesterification column is supplied with a further stream comprising virtually pure alkyl alcohol, the introduction site for which is beneath the introduction site for the dialkyl carbonate-containing alkyl alcohol stream.

6. Process for preparing dialkyl carbonate according to any of Claims 1 to 5, **characterized in that** the cyclic alkylene carbonate used is ethylene carbonate or propylene carbonate, and the alkyl alcohol used is methanol or ethanol.

7. Process for preparing dialkyl carbonate according to any of Claims 1 to 6, **characterized in that** the temperature in the reaction zone(s) is in the range from 20 to 200°C and the pressure is in the range from 0.2 to 20 bar.

## Revendications

1. Procédé pour la préparation continue de carbonate de dialkyle de formule
(R¹O)₂CO,
dans laquelle R¹ représente un C₁-C₄-alkyle linéaire ou ramifié, ainsi que d'alkylèneglycol de formule
HO-R²-OH
dans laquelle R² représente C₂-C₄-alkylène, par transestérification de carbonate d'alkylène cyclique avec un alcool alkylique de formule
R¹OH
dans laquelle R¹ a la signification ci-dessus,
en présence d'un catalyseur, **caractérisé en ce que** la chaleur de condensation de la colonne de traitement de l'alkylèneglycol est récupérée et utilisée directement ou indirectement pour l'évaporation des flux introduits dans la colonne de transestérification, qui contiennent de l'alcool alkylique.

2. Procédé pour la préparation de carbonate de dialkyle selon la revendication 1, **caractérisé en ce que** la chaleur de condensation de la colonne de traitement de l'alkylèneglycol est utilisée pour le préchauffage de l'alimentation de la colonne de traitement de carbonate de dialkyle ou pour l'évaporation de l'alcool alkylique pour la colonne de transestérification, de manière particulièrement préférée, cette chaleur est utilisée pour l'évaporation de l'alcool alkylique pur et/ou de l'alcool alkylique contenant du carbonate de dialkyle pour la colonne de transestérification.

3. Procédé selon la revendication 1 ou 2, **caractérisé en ce que** la colonne de transestérification contient une zone d'épuisement qui est disposée sous la zone de réaction.

4. Procédé pour la préparation de carbonate de dialkyle selon l'une quelconque des revendications 1 à 3, **caractérisé en ce que** la transestérification dans au moins une colonne de transestérification est réalisée en présence d'un catalyseur à contre-courant de manière telle que le carbonate d'alkylène est introduit dans la partie supérieure de la colonne et un alcool alkylique contenant du carbonate de dialkyle, dont la teneur en carbonate de dialkyle est située à 0,2 jusqu'à 30% en poids, est introduit dans la partie centrale ou inférieure de la zone de réaction de ladite au moins une colonne de transestérification.

5. Procédé pour la préparation de carbonate de dialkyle selon la revendication 4, **caractérisé en ce que** la colonne de transestérification est alimentée en un autre flux contenant de l'alcool alkylique quasiment pur, dont le site d'introduction est disposé sous le site d'introduction du flux d'alcool alkylique contenant du carbonate de dialkyle.

6. Procédé pour la préparation de carbonate de dialkyle selon l'une quelconque des revendications 1 à 5, **caractérisé en ce qu'**on utilise, comme carbonate d'alkylène cyclique, du carbonate d'éthylène ou du carbonate de propylène et, comme alcool alkylique, du méthanol ou de l'éthanol.

7. Procédé pour la préparation de carbonate de dialkyle selon l'une quelconque des revendications 1 à 6, **caractérisé en ce que** la température de la/des zone(s) de réaction est située dans la plage de 20 à 200°C et la pression dans la plage de 0,2 à 20 bars.
